# EUROPEAN PATENT APPLICATION

(11) **EP 4 260 887 A1**
(43) Date of publication of application: **18.10.2023**
(21) Application number: 22168386.5
(22) Date of filing: 14.04.2022
(51) Int. Cl.: A61M 5/20, A61M 5/315

(54) **ELECTRONIC MODULE AND AUTOINJECTOR**

(71) Applicant: medmix Switzerland AG, 9469 Haag (Rheintal) (CH)
(72) Inventor: Cattermole, David, Warwick (GB); O'Hare, Aidan, Coventry (GB); Jones, Matthew, Warwick (GB); Marsh, William, Stratford-upon-Avon (GB)
(74) Representative: Manitz Finsterwald Patent- und Rechtsanwaltspartnerschaft mbB

(57) **Abstract**

An electronic module (27), the electronic module (27) being integrated with or connectable to an autoinjector (1), wherein the electronic module (27) is configured to detect and to monitor a dose administering process with the autoinjector (1) or parameters related thereto and to provide a user of the autoinjector (1) with feedback on the conducted dose administering process or parameters related thereto.

## Description

The present invention relates to an electronic module connectable to an autoinjector and to an autoinjector for drug administering with such an electronic module.

Autoinjectors are well known and widely used by patients for self-administered drug injection. However, it has been found that there exists a desire for a highly functional and reliable surveillance of the drug administering process. Such a surveillance can not only be relevant for the patient themselves but also in particular during clinical tests to monitor the injection behaviour of the test subjects reliably.

Therefore, it is an object of the invention to provide an electronic module configured to provide a highly functional and reliable drug surveillance of the drug administering process with an autoinjector and for an autoinjector with such a surveillance configuration.

This object is solved by the subject matters of independent claim 1. Preferable developments for such configurations are given in the dependent claims.

An electronic module in accordance with the present invention is configured to detect and to monitor a dose administering process with the autoinjector and/or of parameters related thereto and to provide a user of the autoinjector with feedback on the conducted dose administering process and/or parameters related thereto. Said electronic module is either integrated with an autoinjector non-releasably or connectable to a corresponding autoinjector for self-administered drug injection.

The detection and monitoring of the dose administering process and/or at least of parameters related thereto allows a quite functional and reliable surveillance of the drug administering process. Providing the user with feedback can include feedback information related to the completion and/or success of the administering process. This information can be provided to a user directly during the administering process to guide the user and/or immediately after the end of the drug administering process to inform the user of the success of the administering process. Furthermore, the feedback information can be provided in the form of signals forwarded to a portable device of the user and/or to a server for storing the information for later evaluation. Thus, the electronic module allows highly flexible and reliable surveillance of the drug administering process and, thus, of the administering process with the autoinjector.

Preferably, the autoinjector comprises a housing, a cartridge or a syringe filled with a drug to be dispensed and positioned within the housing, a plunger positioned within the cartridge or syringe, a driver positioned within the housing and coupled to the plunger. The driver comprises a drive chassis with a plunger rod and a locked drive spring, wherein the locked drive spring is acting between the housing and the drive chassis and, thus, the plunger rod for generating the force necessary to move the plunger for dispensing the drug. Furthermore, the autoinjector comprises a needle guard provided axially movable within the housing and configured to release the locked drive spring of the driver for dispensing the drug upon axial movement of the needle guard.

The above structural configuration for an autoinjector is quite simple and, thus, cost-efficient, while still being highly reliable and secure in use.

Preferably, the electronic module comprises a detection unit and a notification unit. The detection unit is configured to monitor, i.e. to surveil, one or more moving parts of the autoinjector and to generate information about an administering process of the autoinjector and/or parameters related thereto based on an operating state of at least one component of the autoinjector. The notification unit is configured to generate and to output a notification signal including or indicating the generated information about the administering process and/or the parameters related thereto.

Such a configuration was found to be quite favorable for implementing the surveillance or detection, respectively, and the notification in an electronic module.

Further preferably, the detection unit comprises a photo-reflector sensor configured to monitor the movement of at least one component of the autoinjector, in particular of a drive chassis of the autoinjector, during the administering process.

A photo reflector sensor depicts a quite reliable and cheap option for implementing the detection functionality.

In such a configuration, the drive chassis comprises preferably at least one optical pattern which is monitored by the photo-reflector sensor for monitoring the dispensing movement of the plunger rod.

This implementation of the movement detection was found to be quite reliable and cost-efficient.

Alternatively, the detection unit comprises preferably a time of flight sensor configured to monitor the movement of at least one component of the autoinjector, in particular of a plunger rod of the autoinjector, optionally wherein the plunger rod is a part of a drive chassis of the autoinjector, during the administering process.

Such a configuration is also a quite cost-efficient but highly reliable option for implementing the detection functionality.

In such a configuration, the time of flight sensor is preferably an optical time of flight sensor and the plunger rod comprises a reflective surface which is monitored by the optical time of flight sensor for monitoring the dispensing movement of the plunger rod.

This specific implementation was found to be of specific reliability and cost efficiency.

Further alternatively, the detection unit comprises a directional microphone, in particular a micro-electromechanical system microphone, configured to detect a signal sound generated by at least one component of the autoinjector, in particular of a drive chassis of the autoinjector, during the administering process.

Such a configuration is a further quite cost-efficient but highly reliable option for implementing the detection functionality.

In such a configuration, the drive chassis comprises preferably at least one acoustically active section, in particular a clicker protrusion, generating an acoustic signal upon movement of the drive chassis in the proximal direction, such that the directional microphone can monitor the movement of the plunger rod via the generated acoustic signal.

This specific implementation was found to be of specific cost efficiency.

Further alternatively, the detection unit comprises preferably at least one frangible circuit element configured to be broken when at least one component of the autoinjector, in particular a drive chassis of the autoinjector, reaches a specific position during the administering process, to monitor the administering process.

Such a configuration is a further quite cost-efficient but highly reliable option for implementing the detection functionality.

Preferably, the notification unit comprises an active wireless communicator configured to transmit information actively wirelessly to a user device like a smart phone or similar.

Providing a wirelessly communicating notification unit allows to implement highly flexible and connected configurations.

In such a configuration, the active wireless communication can be based on the "cellular" communication technology SigFox.

This communication technology was found to be particularly suitable for implementing the active wireless communication of the electronic module. In particular such a "cellular" communication technology allows the electronic module to transmit data directly to a remote server, without the need for a local intermediate device to relay the information.

Alternatively, the active wireless communication can be based on the "cellular" communication technology NB-IoT.

This communication technology depicts suitable alternative of "cellular" communication technology and thus was also found to be particularly suitable for implementing the active wireless communication of the electronic module. However, also other "cellular" communication technologies could be used in the electronic module.

Further alternatively, the active wireless communication can be based on Bluetooth.

Bluetooth based systems are well known and cheaper than the two above discussed "cellular" communication technologies.

Alternative to the above described active wireless communicator, the notification unit can comprise a passive wireless communicator configured to transmit information passively wirelessly to a user device like a smartphone or similar.

Such passive wireless communicators in principle have the advantage over active wireless communicators that they do not need a separate power supply to be operated. This allows to reduce the size and costs of the electronic module.

In such a configuration, the passive wireless communication is preferably based on NFC (near-field communication), in particular with an RFID chip and an antenna.

This specific implementation for the passive wireless communicator was found to be a highly robust and reliable option for implementing the passive wireless communication in the electronic module.

Preferably, the notification unit comprises an optical notifier configured to directly display information to a user of the autoinjector.

Such an optical notifier allows to provide feedback to a user of the autoinjector directly without the necessity of any further electronic devices. This direct optical feedback can be given not only after the completion of the drug administering process but also during the administering process to guide the user.

In such a configuration, the optical notifier can include at least one, in particular several, notification LED.

Notification LEDs have been found to depict a quite cheap and suitable option for implementing the optical notifier.

In configurations with an active wireless communicator and/or an optical notifier, the electronic module comprises preferably a micro-controller and a battery. Said micro-controller is configured to receive the information about the administering process from the detection unit, to process the received information and to control the operation of the notification unit to output a user feedback based on the processed information.

Such a configuration is believed to be quite reliable but still simple and, thus, cheap to implement.

Further preferably, the electronic module comprises preferably a, in particular mechanical, wake switch. Said wake switch is coupled to the micro-controller and configured to switch the operation state of the micro-controller between an ON state and an OFF state.

The provision of such a wake switch allows to increase the operable lifetime of the electronic module as the electronic components of the electronic module is switch off as long as the electronic module or the autoinjector, respectively, is not in use. Thus, the depletion of the provided power supply is minimized during non-usage of the autoinjector and, thus, of the electronic.

In such a configuration, the wake switch is preferably configured not to be operated by a user of the autoinjector but automatically during the usage of the autoinjector, in particular either by the needle guard or by the drive chassis with the plunger rod.

This implementation allows on the one hand a reliable prevention of unintentional actuation of the components of the electronic module and thus of an unintentional depletion of the power supply. On the other hand, it is ensured that the user cannot miss the activation of the electronic module in time to survey the drug administering process.

Preferably, the electronic module is provided as integral component of the autoinjector non-releasably coupled to the other components of the autoinjector.

Such an implementation results in quite reliable surveillance of the administering process without the necessity of handling the autoinjector and the electronic module as two independent but interacting devices. Thus, the handling is simplified substantially.

In such a configuration, the components of the electronic module are preferably positioned within the housing of the autoinjector.

This allows to implement a quite compact and robust overall configuration.

In particular, the electronic module is positioned side by side with the components of the autoinjector.

Such a configuration allows to prevent an increase in the length of the autoinjector as compared to autoinjectors not provided with the electronic module.

Alternatively, the electronic module can be provided as separate add-on module configured to be coupled releasably to the autoinjector.

Thus, it would be possible to use one single electronic module together with various autoinjectors one after another without the necessity of disposing the electronic module. This allows not only a quite environmentally friendly but also relatively cheap surveillance of various administering processes with various autoinjectors.

In such a configuration, the electronic module comprises preferably a sealing membrane positioned between the electronic module and the autoinjector in a state in which the electronic module is coupled to the autoinjector.

Such a sealing membrane allows to prevent the intrusion of dirt or water from the environment of the autoinjector into the connection between the electronic module and the autoinjector, and thus possible problems related to such pollution.

In particular, the said sealing membrane is made of material that is transparent for specific light, in particular IR light, such that an optical time of flight sensor can be used for monitoring the administering process through the sealing membrane.

It was found that the usage of such an optical time-of-flight with the add-on on electronic module would depict a quite reliable and cost-efficient implementation, while the transparent sealing membrane ensures the operability of the electronic module.

Preferably, such an add-on electronic module comprises an attachment switch configured to detect the attachment of the electronic module to the autoinjector.

Thus, the electronic module is configured to detect automatically the connection to the corresponding autoinjector, which simplifies the handling sufficiently.

In particular, the autoinjector provided for the usage with such add-on electronic module is provided with an NFC tag containing information about the drug stored within the autoinjector.

Such an implementation allows an automatic readout of the information about the drug stored within the autoinjector and, thus, allows to increase the functionality of the electronic module.

In such a case, the electronic module comprises preferably an NFC reader configured to automatically read the information stored within the NFC tag.

Thus, the information about the drug stored within the autoinjector can be read out automatically by the electronic module.

Preferably, at least one component of the autoinjector comprises a barcode or QR code containing information about the drug within the autoinjector and configured to be read by a user device like a smartphone.

Such a barcode or QR code is a quite simple but efficient way to provide information about the autoinjector to a user device.

Preferably, an add-on electronic module comprises a mechanical coding feature only allowing the attachment of the electronic module to autoinjectors provided with a matching mechanical coding feature.

Thus, a dedication of an electronic module to specific autoinjectors is implemented in a quite cost-efficient but reliable manner.

Preferably, the electronic module is configured to be positioned or is positioned at a distal end of the autoinjector axially behind the other components of the autoinjector.

Thus, the width of the autoinjector is not increased by the electronic module. Accordingly, the handling of the autoinjector is less influenced by the provision of the electric module.

Preferably, the autoinjector is a single use fixed dose autoinjector.

Such autoinjectors are structurally quite simple and, thus, quite cheap.

Alternatively, the autoinjector is a single use variable dose autoinjector.

Such autoinjectors are more flexible in use then fixed dose autoinjectors.

Further alternatively, the autoinjector is a multiple use fixed dose autoinjector.

Such autoinjectors are structurally quite simple but with the multiple use configuration more environmentally friendly than the single-use autoinjectors.

Finally, the autoinjector can be a multiple use variable dose autoinjector.

Such an implementation provides the most flexibility and environmental friendliness.

Preferably, the electronic module is configured to detect and to monitor the dose administering process with the autoinjector or parameters related thereto based on a movement status of one or more moving parts of the autoinjector or based on a position of one or more moving parts of the autoinjector.

Such implementations were found to result in quite reliable results for the surveillance of the administering process.

Alternatively, the electronic module is configured to detect and to monitor the dose administering process with the autoinjector or parameters related thereto based on both of a movement status and a position of one or more moving parts of the autoinjector.

The combined movement and position detection results in the most reliable results for the surveillance of the administering process.

In such a configuration, the electronic module is preferably configured to detect and to monitor the dose administering process with the autoinjector or parameters related thereto based on the movement status and the position of one single moving part of the autoinjector, in particular a drive chassis with the plunger rod.

The monitoring of one single moving part it is rather simple and thus cost-efficient, while allowing still reliable surveillance of the administering process. The drive chassis with the plunger rod is the component identified to depict the best option for such as single part of the autoinjector as its movement is directly connected to the administering process.

Preferably, the electronic module is configured to wait a specific period of time before finally determining the end of the dose administering process after no further change in any one of the monitored parameters is determined any more.

Such a time delay was found to be as simple option for ensuring that the administering process is completed.

In particular, the specific period of time is 1, 2, 3, 5 or 10 seconds.

Said values for the specific period of time have been found to be specifically suitable.

Preferably, wherein an outer contour of the autoinjector together with the electronic module is flush seen along the longitudinal axis of the autoinjector.

It was found that such a flush outer contour would result in an improved handling of the overall system.

Exemplary embodiments and functions of the present intervention are described in the following in conjunction with the appending drawings, wherein:
- FIG. 1: shows a cross-sectional view of an autoinjector with a basic injection configuration of an exemplary single-use fixed-dose autoinjector to be used with the electronic module of the present invention;
- FIG. 2A: shows a cross-sectional view of the autoinjector of FIG. 1 as delivered to user;
- FIG. 2B: shows a cross-sectional view of the autoinjector of FIG. 1 directly before drug delivery;
- FIG. 2C: shows a cross-sectional view of the autoinjector of FIG. 1 after completion of the administering process;
- FIG. 3: shows a cross-sectional view of a first exemplary embodiment for an autoinjector with an electronic module according to the present invention;
- FIG. 4: shows enlarged and turned sectional view of the autoinjector of FIG. 3;
- FIG. 5A: shows further details of the autoinjector of FIGS. 3 and 4 before drug dispensing;
- FIG. 5B: shows further details of the autoinjector of FIGS. 3 and 4 during drug dispensing;
- FIG.6: shows diagrams describing the function of the wake switch of the autoinjector of FIGS. 3 to 5B;
- FIG. 7: shows a cross-sectional view of a second exemplary embodiment for an autoinjector with an electronic module according to the present invention;
- FIG. 8: shows an enlarged and turned sectional view of the autoinjector of FIG. 7;
- FIG. 9: shows a cross-sectional view of a third exemplary embodiment for an autoinjector with an electronic module according to the present invention;
- FIG. 10A: shows a cross-sectional view of a further exemplary embodiment for an autoinjector, configured to be connected with an add-on electronic module in accordance with the present invention;
- FIG. 10B: shows a cross-sectional view of the autoinjector of FIG. 10A coupled with an exemplary add-on electronic module in accordance with the present invention;
- FIG. 11: shows an enlarged and turned view of the electronic module of FIG. 10B;
- FIG. 12: shows symbolically two exemplary wireless drug identification configurations in accordance with the present invention;
- FIG. 13: shows symbolically a further exemplary wireless drug identification configuration in accordance with the present invention; and
- FIG. 14: shows an exemplary embodiment of a mechanical drug identification configuration.

In this disclosure, the term "distal part/end" refers to the part/end of the device, or the parts/ends of the components or members thereof, which in accordance with the use of the device, is located the furthest away from a delivery/injection site of a patient. Correspondingly, the term "proximal part/end" refers to the part/end of the device, or the parts/ends of the members thereof, which in accordance with the use of the device is located closest to the delivery/injection site of the patient.

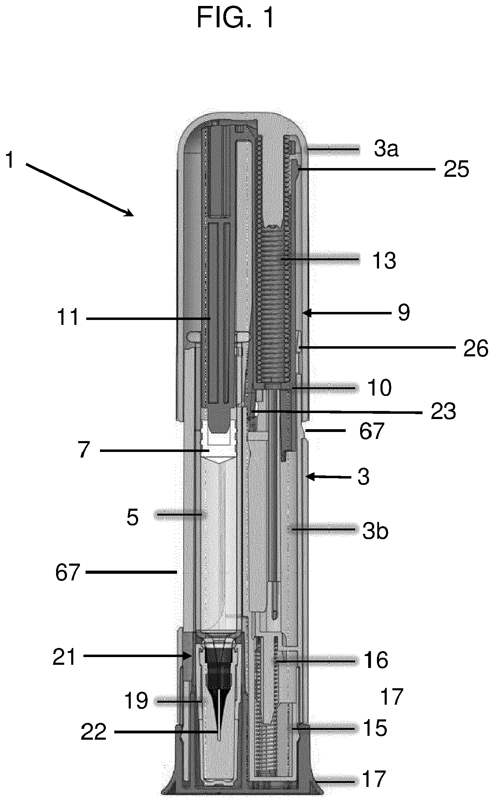

The present disclosure of administering process surveillance is applicable with a number of autoinjectors. One possible autoinjector is a pen-type designed autoinjector with the basic injection configuration as illustrated in FIG. 1.

FIG. 1 shows an exemplary embodiment of an autoinjector 1 configured to be supplemented by or configured to be used together with an electronic module 27 according to the present invention.

The autoinjector 1 comprises a housing 3 formed of an outer body 3a and an inner body 3b. A cartridge or syringe 5 filled with a drug to be administered is positioned within the housing 3. A plunger 7 for dispensing the drug from the cartridge or syringe 5 is positioned within the cartridge or syringe 5. Furthermore, a driver 9 is positioned within the housing 3 and coupled to the plunger 7. Said driver 9 comprises a drive chassis 10 with plunger rod 11 and a locked drive spring 13 acting between the housing 3 and the drive chassis 10 with the plunger rod 11 for generating the force necessary to move the plunger 7 through the cartridge or syringe 5 for dispensing the drug. Moreover, the illustrated autoinjector 1 comprises a needle guard 15 movable against the force of a needle guard spring 16 from a proximal end of the autoinjector 1 in the distal direction into the housing 3. Said needle guard 15 is coupled to the drive chassis 10 in such a manner that a distal movement of the needle guard 15 releases the locked drive spring 13 resulting in a proximal movement of the plunger rod 11 and, thus, of a dispensing movement of the plunger 7 along the cartridge or syringe 5.

For the sake of security, the autoinjector 1 further comprises a cap 17 with a rigid needle shield 19 connected to the proximal end of the autoinjector 1. This cap 17 not only shields the needle 22 but also prevents an distal movement of the needle guard 15.

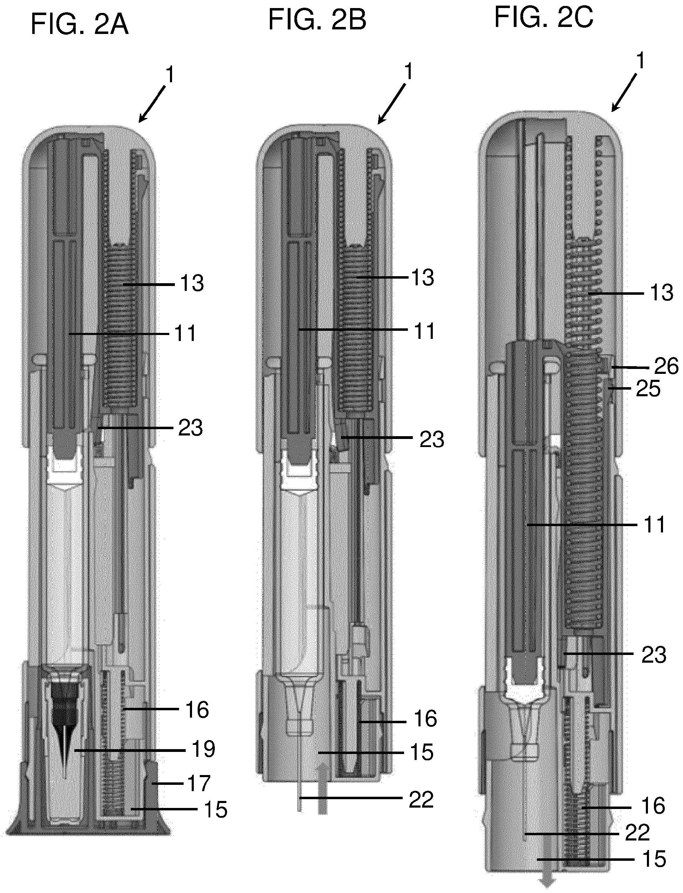

In the following, the basic function of the illustrated autoinjector 1 will be described with reference to FIGS. 2A to 2C.

As can be seen in FIG. 2A, in the delivery state of the autoinjector 1 the cap 17 is positioned on the proximal end of the autoinjector 1. The rigid needle shield 19 of this cap 17 encloses a needle assembly 21 with the needle 22 of the cartridge or syringe 5. The drive chassis 10 with the plunger rod 11 and the drive spring 13 are in an initial state, in which the drive spring is loaded and locked via a locking element 23 being engaged with the housing 3. Thus, the autoinjector 1 is in a prefilled and pre-loaded status.

For the drug administering, the cap 17 together with the needle shield 19 is removed from the proximal end of the autoinjector 1. Then, the autoinjector 1 is pressed with the needle guard 15 at its proximal end against an injection side (not illustrated). This results in a distal movement of the needle guard 15 with respect to the housing 3, while the needle 22 of the needle assembly 21 penetrates the injection site. Near a distal end position of the needle guard 15, the needle guard 15 disengages the locking element 23 of the drive chassis 10 from the housing 3 and, thus, releases the drive spring 13. Exactly this situation is illustrated in FIG. 2B.

The released drive spring 13 pushes the drive chassis 10 with the plunger rod 11 in the proximal direction, which results in a corresponding movement of the plunger 7 and, thus, in a dispensing of the drug from the cartridge or syringe 5 through the needle 22 into the injection site. After this dispensing, the autoinjector 1 is removed from the injection site. This results in the needle guard spring 16 pushing the needle guard 15 in the proximal direction such that the needle guard 15 encloses the needle 22. In this extended state, an acoustically active section 25 of the drive chassis 10 engages with an end of dose click protrusion 26 of the housing 3 generating an end of dose click and a locking the movement of the components of the driver 9 with respect to the housing 3. Moreover, the locking element 23 of the drive chassis 10 engages with the needle guard 15 preventing a further distal movement of the needle guard 15 with respect to the housing. This finally locked state is illustrated in FIG. 2C.

In this connection it should be noted that the drive chassis 10 is arranged linearly moveable within the housing 3. The drive chassis 10 comprises a dispensing limb forming the plunger rod 11 and a trigger limb within which the drive spring 13 is arranged. The plunger rod 11 is arranged at a proximal end of said dispensing limb and the locking element 23 is arranged extending proximally from said trigger limb. The trigger limb and the dispensing limb are arranged in parallel to one another respectively at least essentially in parallel to one another and are connected to one another, preferably are integrally formed in one piece, at a respective distal end of the dispensing limb and the trigger limb. This shape of drive chassis 10 enables a particularly compact design of the autoinjector 1.

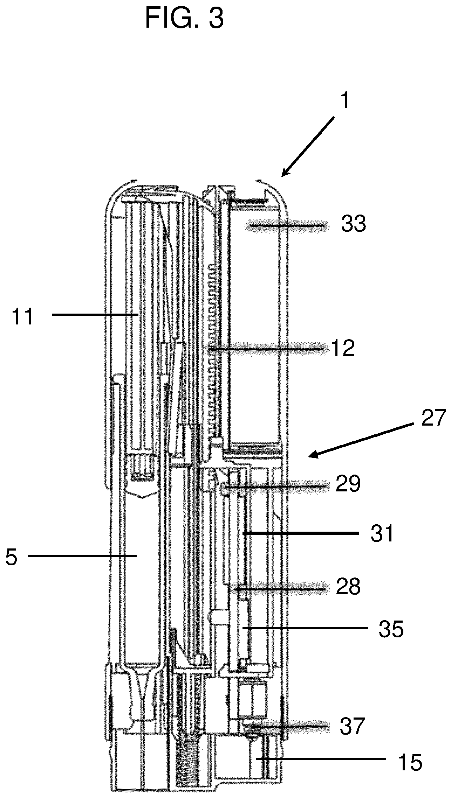

In the following, a first exemplary embodiment for an autoinjector with an integrated electronic module in accordance with the present invention will be described with reference to FIGS. 3 to 6.

The basic injection configuration of the here illustrated autoinjector 1 is in principled identical with the one of the autoinjector 1 described with reference to FIGS. 1 to 2C.

The above described autoinjector 1 is merely supplemented with an exemplary electronic module 27 in accordance with the present invention. Said electronic module 27 is positioned side by side with the above described components of the autoinjector 1 within the housing 3, in particular within the outer body 3a of the housing 3.

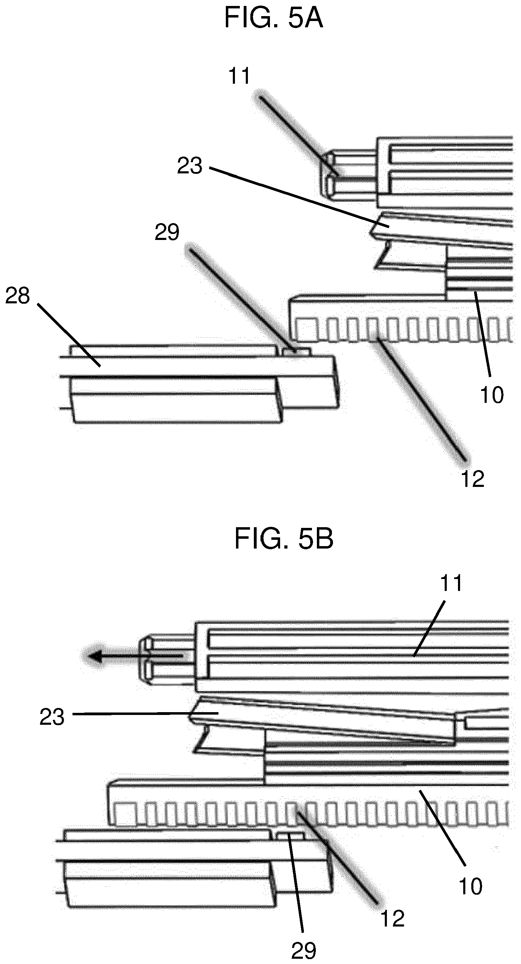

The electronic module 27 comprises a printed circuit board arrangement (PCBA) 28 including a detection unit with a photo-reflector sensor 29 and a micro-controller 31 as well as a power supply 33 in the form of a battery, in particular of a standard AAA size battery (preferably either alkaline or LiMnO₂). The micro-controller 31 is coupled to an active wireless communicator 35 in the form of an NB-IoT module of a notification unit of the electronic module 27. Furthermore, a wake switch 37 (described later in more detail) is provided in the proximal end of the housing 3 and coupled to the micro-controller 31. Finally, also an optical notifier in the form of a notification LED (not illustrated) is connected to the micro-controller 31. The drive chassis 10 is provided, in particular printed, with an optical pattern 12 in the form of a printed coded track with sections of differing reflectivity. This is illustrated in FIGS. 5A and 5B, where FIG. 5A shows the relative positioning between the optical pattern 12 and the photo-reflector sensor 29 immediately before the release of the drive spring 13 and FIG. 5B shows the relative positioning between the optical pattern 12 and the photo-reflector sensor 29 at a specific point of time during the dispensing movement of the drive chassis 10 with the plunger rod 11.

The photo-reflector sensor 29 is positioned such that the region of the drive chassis 10 with the optical pattern 12 passes the photo-reflector sensor 29 during a dispensing movement of the drive chassis 10 with the plunger rod 11 in the proximal direction. Thus, the photo-reflector sensor 29 is configured to monitor the dispensing movement of the plunger rod 11 with respect to the housing 3. For this, the photo reflector sensor 29 is coupled non-movably to the housing 3. The micro-controller 31 is configured to receive varying brightness information corresponding to the movement of the drive chassis 10 with respect to the photo-reflector sensor 29 and to process the received information. In particular, the micro-controller 31 is configured to determine both the present position of the drive chassis 10 with the plunger rod 11 within the housing 2 and the present movement status of the drive chassis 10 with the plunger rod 11 for evaluating the current state of the administering process. However, the micro-controller 31 can also be configured to determined only one of the present position of the drive chassis 10 with the plunger rod 11 within the housing 2 and the present movement status of the drive chassis 10 with the plunger rod 11 for evaluating the current state of the administering process, if desired. The micro-controller 31 is further configured to control the operation of the various notification units of the electronic module 27, here in particular of the NB-IoT module 35 and of the notification LED to output a user feedback based on the processed information.

In particular, the notification LED is illuminated and switched off in a particular pattern to inform the user of the autoinjector 1 of the status of the administering process. At the same time, the NB-IoT module 35 transmits wirelessly electronic information about the administering process to an external server, where this information is stored.

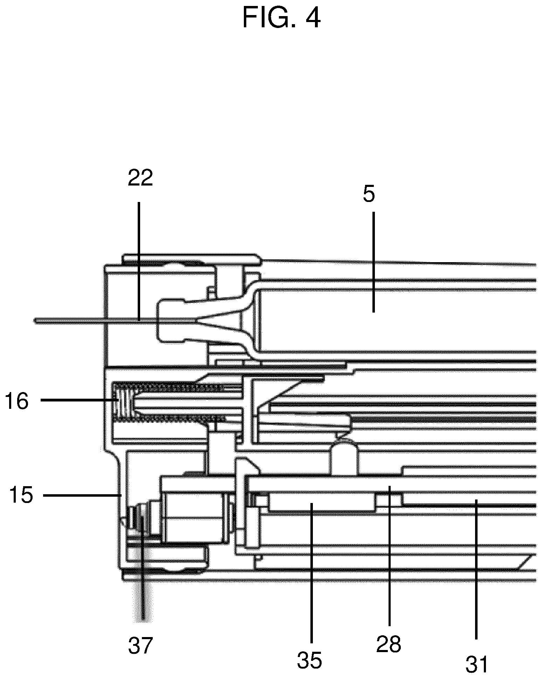

To extend the service life of the electronic module 27, the micro-controller 31 is configured to be in an inactive sleep-state until the wake switch 37 is activated. For this, the wake-switch 37 is positioned in such a manner that it is activated only through a distal movement of the needle guard 15 immediately before the release of the drive spring 13 as can be seen in FIG. 4. The wake switch 37 is a mechanical wake switch 37 due to the minimum energy consumption of such a mechanical switch in comparison to accelerometers or thereto similar components.

The micro-controller 31 is not only configured to notice the activation of the wake switch 37 but also its deactivation thereof upon a proximal movement of the needle guard 15 and a thereto related release of the wake switch 37. In particular, the micro-controller 31 is configured to determine the period of time during which the wake switch 37 is activated and to compare this period of time with a predetermined period of time expected to be necessary for a full administering process. Accordingly, the micro-controller 31 is configured to not only detect and monitor the dose administering process based on the movement of the drive chassis 10 with the plunger rod 11 but also based on the movement of the needle guard 15.

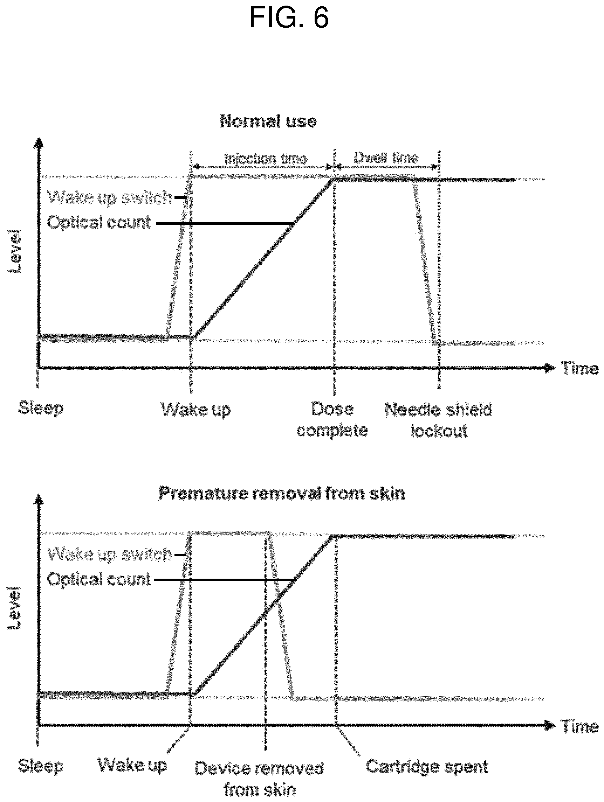

As for example illustrated in FIG. 6, a full administering process would at least require a specific predetermined injection time and a further dwell time from the full activation of the wake switch 37 (see the above diagram). If the wake switch 37 is deactivated before the expiration of this predetermined period of time by a removal of the autoinjector 1 from the injection site and, thus, by a proximal movement of the needle guard 15, the micro-controller 31 determines an incomplete administering process, although the drive chassis 10 with the plunger rod 11 finishes its whole dispensing movement.

In other words, the use of the needle guard 15 to actuate the wake switch 37 offers the benefit in being able to detect premature removal of the autoinjector 1 from the injection site before dose delivery has completed.

In other words, during normal operation, the wake switch 37 is actuated before movement of the plunger rod 11 commences, and the wake switch 37 is not released until after movement of the plunger rod 11 has ceased. When the autoinjector 1 is lifted away from the injection site the needle guard spring 16 will displace the needle guard 15 proximally, into its extended position. The wake switch 37 will register a change in state since the needle guard 15 will no longer be in interference with the wake switch 37. Therefore, if movement of the plunger rod 11 continues after the wake switch 37 is released this indicates premature removal of the autoinjector 1. In this case, the event may be recorded as an error within the micro-controller 31 to be transmitted alongside the dispense time and drug information. This information may be subsequently used to mitigate future premature removal either through user training or other intervention.

Such a functionality is of particular relevance when the micro-controller 31 is provided with further information about the autoinjector 1 like the two above referenced time periods. In the present configuration, in which the electronic module 27 is integrated into the autoinjector 1, these and also other information like the specific drug within the cartridge or syringe 5 can be directly stored in a memory connected to the micro-controller 27.

As indicated above, a full administration of the drug could require a specific dwell time. Thus, if for example the optical notifier LED is used to guide the user through the administering process, the micro-controller 31 of the electronic module 27 could wait a specific period of time before indicating the end of the dispensing process after the monitored component, here the drive chassis 10 with the plunger rod 11, is not moving any more, respectively after the monitored parameter is not changing any more. It has been found that suitable time periods lie between 1 and 10 seconds, depending on the used drug and the structural configuration of the autoinjector 1 itself.

In principle, there are various aspects of a dose administering process which could be detected and monitored by the electronic module 27. In particular, the start of the dose dispensing and the successful completion of the dose dispensing can be determined. Furthermore, the electronic module 27 can be configured to determine a termination of the dispensing process, for example after no dispensing movement or change in the monitored parameter is determined for a specific time going for example from 1 sec to 10 secs after the start of the dose dispensing was determined but the successful end of the dose dispensing has not been determined yet. Also, the specific development of the administering process could be monitored, in the present embodiment in particular based on the movement characteristics of the drive chassis 10 with the plunger rod 11 with respect to the photo-reflector sensor 29 and, thus, with respect to the housing 3.

For this, the photo-reflector sensor 29 comprises an LED for illumination and a photo transistor for detecting the light reflected from the optical pattern 12 on the drive chassis 10. The optical pattern 12 is formed of more reflective and less reflective regions positioned in view of the photo-reflector sensor 29 such that an axial movement of the drive chassis 10 with the plunger rod 11 results in changes in intensity of reflected light determined by the photo transistor. This signal is transmitted to the micro-controller 31.

Such a sensing system provides a relative measure of the distance travelled by the drive chassis 10 with the plunger rod 11 and allows the micro-controller 31 to determine that the plunger rod 11 has translated far enough for full drug delivery. Furthermore, the micro-controller 31 can determine when movement of the drive chassis 10 with the plunger rod 11 has ceased. The final axial position of the plunger rod 11 to the housing 3 at the end of dispense is subject to large tolerance variation, primarily due to this end stop being defined by the plunger 7 at the proximal end of the cartridge or syringe 5. As a result, if a mechanical switch were used to detect end of dispense, the combined tolerance effect of the device tolerances coupled with the switch point variation and PCB mounting tolerances mean such an alternative system would be too imprecise. By detecting both the axial position of the drive chassis 10 with the plunger rod 11 and whether it has ceased moving allows the micro-controller 31 to more reliably identify the end of the dispensing operation even with relatively large mechanical and electronic detection mechanism tolerances.

If the end of dispense is determined through detecting the axial position of the drive chassis 10 with the plunger rod 11 only, the point at which end of dispense feedback is triggered must be nominally arranged to occur before the plunger rod 11 ceases moving. This ensures that, even when the plunger rod 11 comes short of its nominal axial displacement due to component and system tolerances, the end of dispense feedback is still triggered. By also monitoring the continuing movement of the drive chassis 10 with the plunger rod 11, this information can be combined with the relative displacement of the plunger rod 11 and the end of the dispensing process may be only determined when the drive chassis 10 with the plunger rod 11 has advanced far enough and also ceased moving. This improves the accuracy of the timing of the end of the dispensing process and ensures it more closely aligns with the precise moment that dose delivery completes.

Here, the optical pattern 12 is a printed pattern on the drive chassis 10, since minimal changes to the original autoinjector mechanism (see FIGS. 1 to 2C) are preferred. However, functionally, this optical pattern 12 could be also formed by moulded-in features/recesses, laser surface markings, a separate component or other similar techniques. In addition, other sensing technologies, such as coded magnets or capacitance sensors could be employed to provide similar feedback of the plunger rod 11 movement.

In the present embodiment, the successful completion of the dose administering process is determined when the drive chassis 10 with the plunger rod 11 has reached full proximal displacement and has ceased moving. The micro-controller 31 is configured to identify a stall (either due to mechanism or PFS failure), where the plunger rod 11 stops moving completely or moves so slowly that it does not achieve full proximal displacement within a pre-determined time limit. This would be recorded as an error event.

On dispense completion the system attempts data transmission to a remote system (the 'cloud') using the NB-IoT or similar network. Optionally, the data transmission may not start until the wake switch 37 has also been released, indicating that the needle guard 15 has extended as the autoinjector 1 is removed from the injection site. The data transmitted may include variables relating to the dose administering process such as the time since wake, time since dispense completion, time since error state and displacement of the drive chassis 10 with the plunger rod 11, device status flags such as detection of a stall, premature removal, low battery etc. alongside drug specific information such as type, concentration, batch and expiry date, alongside data that can be used to link the device to the patient to which it was prescribed.

The one or more notification LED's, visible to the user through a cut-out, transparent window or thin region in the housing 3, provide visual feedback to the user during use of the autoinjector 1. In particular, different LED flashing patterns and/or colors may be used to indicate the status of the autoinjector 1 to the user. Examples for the status is whether the wake switch 37 is activated, dispense is in progress, the dose has been successfully delivered, an error state has been detected, data transmission is in progress and whether data has been successfully transmitted.

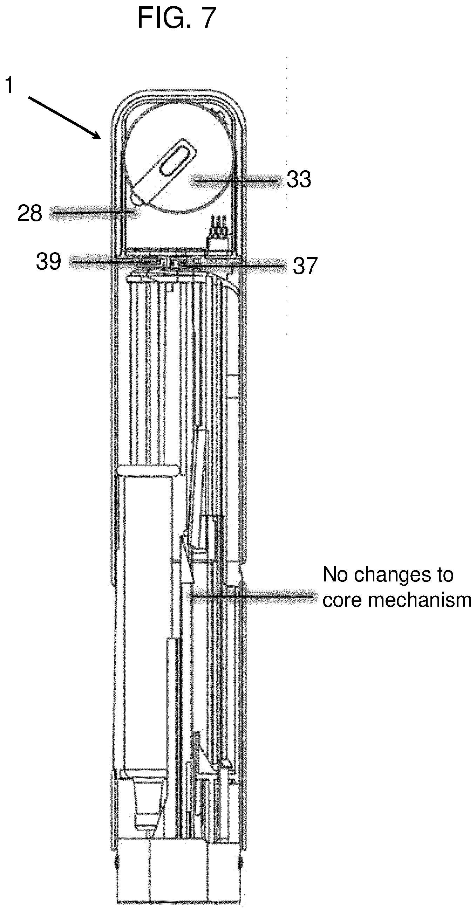

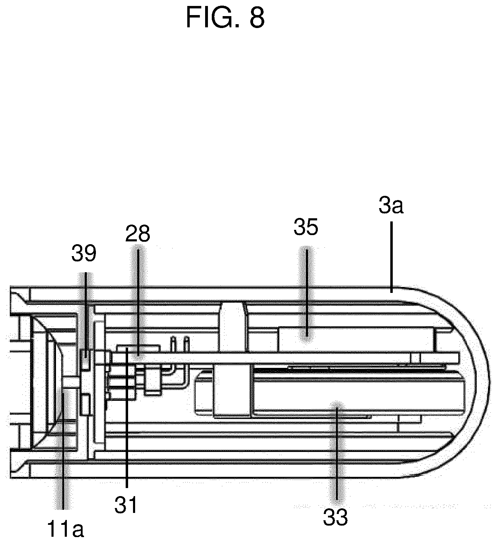

In the following, a second exemplary embodiment of an autoinjector 1 with a corresponding electronic module 27 will be described with reference to FIGS. 7 and 8.

Here, the electronic module 27 is provided as integrated module provided within a distal end of the housing 3 behind the other components of the autoinjector 1. The outer body 3a of the housing 3 is increased in length to accommodate the power supply 33 and other electronics mounted in series with the injection mechanism to facilitate the connectivity features.

No changes are required to the core principles and the majority of the components of the injection mechanism of the autoinjector 1 as illustrated in FIGS. 1 to 2C.

The electronic module 27 in this embodiment substantially comprises a PCBA 28 with a micro-controller 31, an active wireless communicator 35 in the form of a SigFox module, a time of flight sensor 39, a wake switch 37, a notification LED (not illustrated) and a power supply 33.

Due to the reduced space, the power supply 33 is a relatively compact coin cell (high drain CR2032) which have been specifically developed to meet the requirements of the SigFox communication technology. The present cell 33 is mounted using a proprietary coin cell holder. As a result, this integrated option necessitates relatively minor changes in the overall configuration of the autoinjector 1, in particular adding about 27 mm in length and about 1.6 mm in width.

Given the integrated nature of this autoinjector 1, the drug information can be coded directly into a memory of the micro-controller 31 during manufacture and/or assembly. This avoids the need for the electronic module 27 to have the capability to identify different medicaments assembled into the autoinjector 1 and allows this information to be transmitted on completion of the dose administering process.

In the following, the function of the present autoinjector 1 will be described roughly.

Prior to dispense the distal end of the plunger rod 11 interacts with the wake switch 37, holding the wake switch 37 in a depressed state. While this wake switch 37 remains pressed, the electronic module 27 remains in a very low power deep sleep state, maximizing battery life of the autoinjector 1.

As dispense begins, the plunger rod 11 moves proximally, releasing the wake switch 37. This signal initiates the electronic module 27 to bring the micro-controller 31 out of a deep sleep state. In order to achieve the necessary long storage time for these type of devices, the wake switch 37 is a mechanical detect switch and in this case is expected to be a normally closed type (such that when the switch is released, a circuit is completed). In the storage condition therefore, the circuit remains open, so this does not draw quiescent current and allows the micro-controller 31 to remain in a very low energy state until dose dispense starts. Other active sensing options, such as accelerometers draw current themselves, and require the micro-controller 31 to be in a higher power monitoring state throughout the autoinjector 1 storage life prior to drug dispensing.

During dose dispensing the plunger rod 11 continues to translate axially in the proximal direction until the end of dose is reached. It is preferable to detect dose completion in addition to simply the start of a dose administering process to provide positive confirmation that the dose was successfully delivered in full. If the dose administering process is not completed successfully, for example due to mechanism stalling or another adverse event, this can be identified and feedback can be provided to the user or healthcare professional.

A number of different non-contact methods for detecting the end of the administering process are possible. In the present embodiment, a time of flight sensor 39 is used. The present time of flight sensor 39 is an optical time of flight sensors 39 emitting a pulse of infrared light and detecting the time taken for this pulse to be reflected back to an adjacent detector of the sensor 39. The time taken is used to calculate the distance between the sensor 39 and the target.

In this embodiment, the time of flight sensor 39 is positioned to target a distal and reflective surface 11a of the plunger rod 11. During dispense, the distance between the time of flight sensor 39 and the plunger rod 11 increases, until the plunger rod 11 ceases moving on dose dispensing completion. Once the time of flight sensor 39 detects both that the plunger rod 11 is stationary and that it has translated axially far enough to achieve full dispense, the micro-controller 31 records the completion of the dose dispensing process.

This sensing system provides a relative measure of the distance travelled by the plunger rod 11 and allows the micro-controller 31 to determine that the plunger rod 11 has translated far enough and when movement of the plunger rod 11 has ceased.

Alternatively, also a directional microphone 39, in particular a MEMS (micro-electromechanical system) microphone system activated by release of the wake switch 37 can be used instead of the time of flight sensor 39.

When the plunger rod 11 approaches the end of dispense, a characteristic audible click is created by a resilient feature 25 of or on the drive chassis 10 interacting with at least one corresponding feature 26 of the housing 3. Such a resilient feature 25 serves as acoustically active section 25 of the drive chassis 10 and in particular is provided in the form of a clicker protrusion. This is expected to create a specific frequency and amplitude of sound that, via signal processing conducted by either the micro-controller 31 or a separate dedicated micro-processor, can be differentiated from other background noise.

To improve the robustness of the system, multiple distinctive clicks may be generated by the mechanism, all or some of which must be identified by the electronic module 27 before the end of the dose administering process is confirmed. In addition to detecting the end of dose dispensing, the MEMS microphone system could also be used to identify when the needle guard 15 advances, if this is accompanied by another distinguishable sound. This could be used to inform about a correct sequence of operation, in a similar way as the wake switch 27 is employed for premature removal detection as described above. This has the benefit of avoiding the addition of significant measurement related tolerances.

Other possible non-contact methods of detecting the end of dispense (for example accelerometers, ultrasonic sensors, camera-based vision system etc.) are expected to be feasible within this configuration but have not been developed at this stage.

The data transmission to the user and/or other devices in principle is identical to the one described for the first embodiment. The only difference is that compared to the NB-IoT network, the SigFox network is limited in its ability to provide positive confirmation that the transmitted information has been successfully received by the remote system. As a result, the SigFox based electronic module 27 may be configured to repeatedly attempt transmission of the data following dispense for a predefined number of iterations or until the power supply 33 is exhausted to maximize the chance of successfully uploading the data.

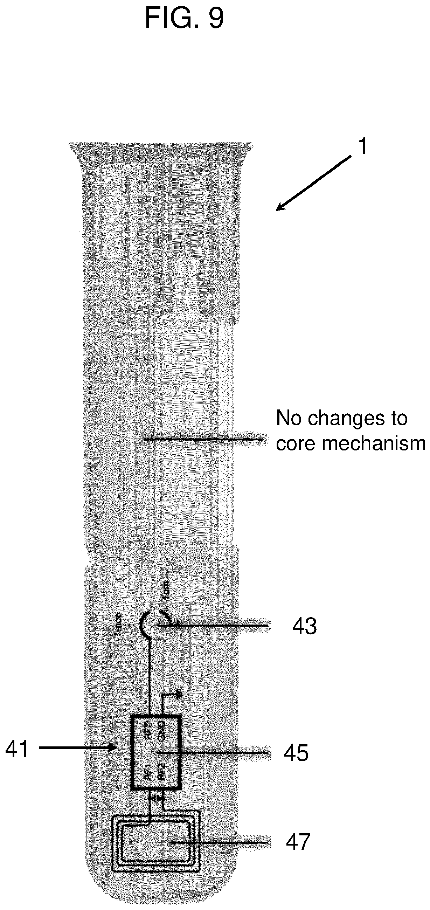

In the following, a further exemplary embodiment for an autoinjector 1 with an electronic module 27 in accordance with the present invention will be described with reference to FIG. 9.

This embodiment requires merely minimal changes to the core mechanism of the autoinjector 1 as described with reference to FIGS. 1 to 2C. Here, the detection or monitoring of the drug administering process and the notification of the user is achieved by an integrated NFC connectivity system 41.

In contrast to the above described "cellular" communication integrated systems, the NFC integrated option does not include a power supply 33. For NFC systems, that utilize RFID (radio-frequency identification) technology, the circuitry integrated into the autoinjector 1 serves as passive wireless communicator and may not require a built-in power supply 33. When a smartphone with NFC capability is brought into close contact with the integrated NFC autoinjector 1, the magnetic field for example created by a smartphone can be used to provide power to the passive NFC tag built into the autoinjector 1.

The illustrated integrated NFC option uses an NFC circuit arranged with a frangible circuit element 43, in particular in the form of a loop of wire, that is broken at the end of the dispensing process by the drive chassis 10. Before use, when a smartphone is brought into close contact with the integrated NFC autoinjector 1, the RFID chip 45 is energized via an antenna 47 and acts as a transponder, providing a specific response to the smartphone to indicate that the autoinjector 1 has not yet been used.

Then, the autoinjector 1 may be activated by a user to dispense the dose without any modification to user steps or specific instructions related to the connectivity system. As the drive chassis 10 with the plunger rod 11 advances proximally during the administering process, it interacts with the frangible loop of wire 43 connected to the RFID chip 45, breaking it as the end of the dispensing process is reached. The breakage of the wire 43 alters the signal that is transmitted by the RFID chip 45 when energized by the smartphone after use so that it can be confirmed that the autoinjector 1 has been used.

It is preferable to arrange the frangible circuit element 43 such that it is broken as near to the end of the dispense stroke of the drive chassis 10 with the plunger rod 11 as possible. In this way, the change of state indicated by the breaking of the element 43 can be used to provide a positive confirmation that the dose was successfully delivered in full.

If the administering process is not completed successfully and the drive chassis 10 with the plunger rod 11 does not proximally translate fully, due to mechanism stalling or another adverse event, the frangible circuit element 43 will not be broken and the autoinjector 1 will not report dose completion.

Drug and/or device information can be programmed into a part of the RFID circuit not affected by the breakage of the frangible circuit element 43, such that this can be read both before and after use of the autoinjector.

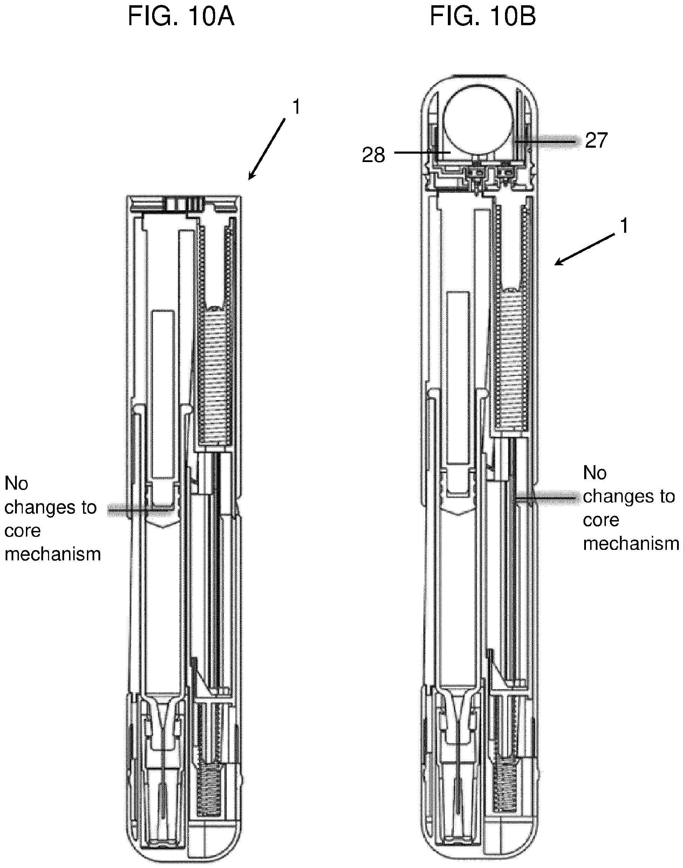

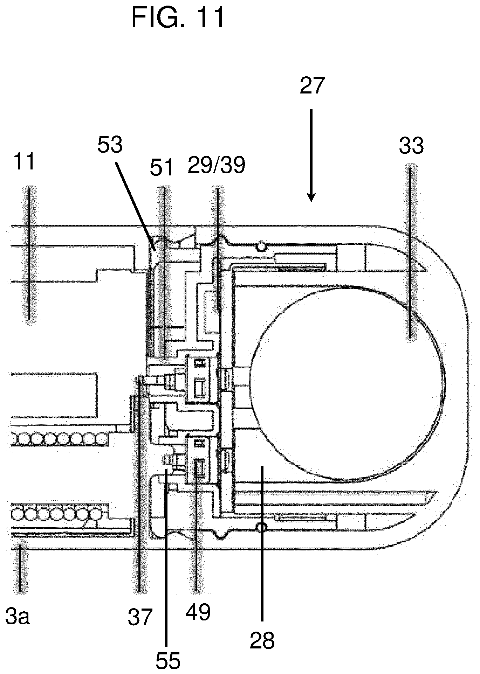

In the following, a further exemplary autoinjector 1 with an electronic module 27 in accordance with the present invention will be described with reference to FIGS. 10A, 10B and 11.

Here, the electronic module 27 is provided as separate add-on module coupled releasably to the distal end of the autoinjector 1. Such an add-on configuration provides the opportunity to re-use the electronic module 27 for multiple doses with multiple different autoinjectors 1.

Only minor modifications to the housing 3 allow a design of the autoinjector 1 to be used both with or without the add-on connected module.

The connected electronic module 27 of the present embodiment substantially comprises a PCBA 28 with a micro-controller 31, a dispense sensor 29 or 39, a wake switch 37, an attachment switch 49, a user notification LED (not illustrated) and a power supply 33. Furthermore, an active wireless communication configuration based on Bluetooth is provided. The lower peak currents involved in Bluetooth transmissions allow for a smaller coin cell to be used as compared to the above described integrated "cellular" options. This allows a quite compact add-on module 27. Such an electronic module 27 can be accommodated within the width and depth of the existing housing 3 of the implementation described with reference to FIGS. 1 to 2C and has a length of approximately 18 mm.

A sealing membrane 51 is provided on the proximal end of the electronic add-on module 27 to prevent ingress of fluids and particles which may affect function of the add-on module 27. The sealing membrane 51 is flexible to enable the wake switch 37 and the attachment switch 49 to operate by deflection of locally thinned or convoluted regions of this sealing membrane 51. In the case where an optical time of flight sensor 39 is employed, the sealing membrane 51 is at least partly also made of material that is transparent for light of specific wavelengths, in particular to IR light, permitting the correct function of the sensor 39.

Said Bluetooth add-on module 27 can be attached and detached from the distal end of the modified housing 3 via demountable clip features 53. The add-on module 27 detects fitment to an autoinjector 1 via the attachment switch 49 which interacts with a localized protrusion 55 on the distal end face of the housing 3. While this attachment switch 49 remains released, indicating that the add-on module 27 is not fitted or incorrectly fitted to an autoinjector 1, the add-on module 27 will remain in a very low power state, conserving battery life.

When the attachment switch 49 is pressed, this indicates that the electronic module 27 has been fitted to an autoinjector 1 and the electronic module 27 may be activated and enter a higher power state to check drug identification, switch operation sequence and conduct a system health check (e.g. confirm battery voltage levels). On fitment to the autoinjector 1, the wake switch 37 is also pressed, via interaction with the distal surface of the plunger rod 11, which is accessible through an opening in the distal end of the housing 3. The height of the localized protrusion 55 on the distal end face of the housing 3 ensures that the attachment switch 49 is pressed before the wake switch 37 as the electronic module 27 is coupled to the autoinjector 1 and released after the wake switch 37 on removal of the electronic module 27. The sequence of switch state change can be used to inform correct fitment of the electronic module 27 to an autoinjector 1.

In addition, a defined sequence or combination of switch press events, and potentially the specific timing between two or more switch state change events, can be used to provide a means of user interaction with the electronic module 27, such as initiating pairing or manual synchronization of stored data with a smartphone. The user could be instructed to press one or more of the available switches in a specific sequence and/or for a certain duration to activate different module functions in the un-coupled state of the electronic module 27.

Similar to the wake switches 37 described above, both the attachment switch 59 and wake switch 37 in this variant are expected to be of a mechanical type to minimize quiescent current drain in storage.

Once the electronic module 27 has confirmed attachment to an autoinjector 1, it monitors the state of the wake switch 37 for indication that dispense has commenced. The wake switch 37 and the dose completion detection (for example the time of flight sensor 39 targeting the plunger rod 11 or MEMS microphone and signal processing to identify the end of dose click) is functionally identical to the arrangement described above with respect to the second exemplary embodiment. Thus, the further function of this embodiment is not described again in detail.

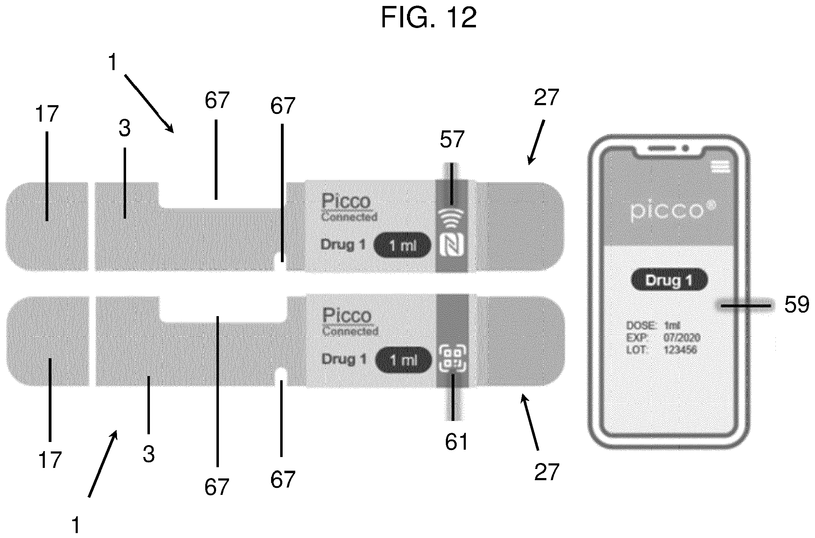

A further key aspect of a connected drug delivery system 1 is the ability to confirm the type and expiry of the drug to be delivered, providing the opportunity to warn or coach the user before an error is made. In particular, given an add-on electronic module 27 there is the potential to fit the electronic module 27 to autoinjectors 1 containing different drugs. For such an add-on module 27, three possible solutions to address this concern are presented in the following with reference to FIGS. 12 to 14.

As illustrated in FIG. 12, according to a first exemplary configuration, an NFC tag 57 is embedded in a label on the housing 3 of the autoinjector 1. This NFC tag 57 contains information about the drug stored within the autoinjector 1. The NFC tag 57 is then read independently by a smartphone 59, in particular prior to the administering process. This data can be stored in the smartphone 59 and then potentially can be combined with dose delivery data provided by the add-on module 27 after completion of drug administering process. Alternatively, a barcode or QR code 61 is printed onto the label to be read by the camera of the smartphone 59. Thus, a user can be informed about specific information of the autoinjector 1 prior to use to guide the following administering process.

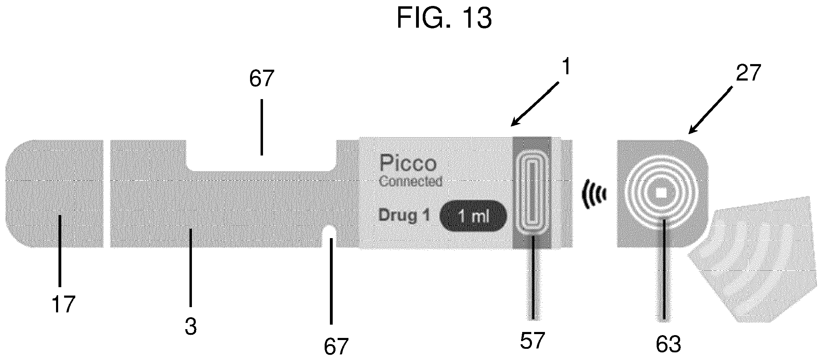

Referring to FIG. 13, an NFC reader 63 can be incorporated into the add-on electronic module 27 to automatically read the NFC tag 57 of the autoinjector 1 as described above. This avoids the need for additional user steps of independently reading the NFC tag 57 with the smartphone 59 and also more reliably couples the drug information with the dose administering process. When the attachment switch 59 is pressed on fitment of the add-on module 27 to the autoinjector 1, the built-in NFC reader 63 is activated to detect the drug information. This provides the opportunity to warn a user if the incorrect combination of add-on module 27 and autoinjector 1 have been connected, either through further Bluetooth communication to the smartphone 59, or via the notification LED's of the electronic module 27 itself. If the drug information shows the medication is acceptable then positive confirmation may also be provided. Following the completion of the drug administering process, the drug information previously read by the add-on electronic module 27 may be combined with any dose event data and this information may be transmitted for example via Bluetooth to the smartphone 59 as a single record.

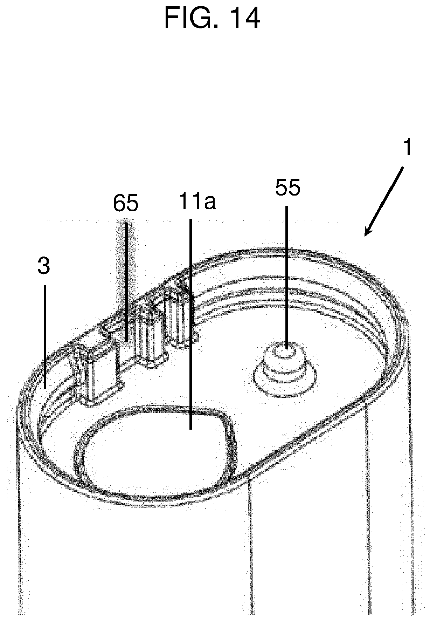

Finally, also mechanical coding features 65 can be incorporated into the interface between the add-on electronic module 27 and the housing 3 of the autoinjector 1 to lock or code certain modules 27 so these can only be assembled to specific autoinjectors 1. An example for such mechanical coding features 65 is illustrated in FIG. 14. For such an arrangement it is considered unlikely that sufficient resolution in the coding system could be realized to achieve unique expiry or batch identification, but drug specific coding is considered feasible.

Referring again to the wireless communication of the electronic module 27, the usage of Bluetooth protocol for communication permits two-way communication and therefore enables the smartphone 59 to confirm successful receipt of the data to the add-on electronic module 27, preventing unnecessary retransmission of data which would reduce battery life.

With the described add-on electronic module 27, following use with one first autoinjector 1, the patient removes the add-on electronic module 27 from the depleted autoinjector 1 and either immediately attaches it to a new autoinjector 1 or stores it independently awaiting fitment to another autoinjector 1, with which the administering process can be repeated.

It is pointed to the fact that the above described autoinjectors 1 are single use fixed dose autoinjectors. Accordingly, only one single dose of a preset amount depending on the internal structural configuration of the autoinjector 1 can be dispensed with these autoinjectors 1.

However, the above described drug administering process surveillance is not restricted to such single use fixed dose autoinjectors 1. If desired, the above described functionalities can also be combined with multiple use fixed dose autoinjectors, allowing the dispensing of multiple preset doses, single use variable dose autoinjectors, allowing the dispensing of a single dose of a user-selectable amount, and multiple use variable dose autoinjectors, allowing the dispensing of multiple doses of a user-selectable amount.

Finally, it is pointed to the finding that configuring the autoinjector 1 and the electronic module 27 in such a manner that the external surface of the overall product in the connected state is flush seen along the longitudinal axis of the autoinjector 1 without any steps or protrusions allows a highly improved handling of the autoinjector 1 with the electronic module 27 by a user. Such a configuration is implemented in all the illustrated embodiments. The cutouts illustrated in FIGS. 12 and 13 depict only windows 67 within the housing 3 but no real recesses perpendicular to the longitudinal axis of the autoinjectors 1.

In the following the basic features related to the present invention are listed with reference to specific aspects of the present invention:
A first aspect of the present invention refers to an electronic module (27) connectable to an autoinjector (1) or an autoinjector (1) with such an electronic module (27), wherein the electronic module (27) is configured to detect and to monitor a dose administering process with the autoinjector (1) and/or parameters related thereto and to provide a user of the autoinjector (1) with feedback on the conducted dose administering process and/or parameters related thereto.

A second aspect of the present invention refers to the electronic module (27) or autoinjector (1) of the first aspect,
wherein the autoinjector (1) comprises: a housing (3); a cartridge or a syringe (5) filled with a drug to be dispensed and positioned within the housing (3); a plunger (7) positioned within the cartridge or syringe (5); a driver (9) positioned within the housing (3) and coupled to the plunger (7), wherein the driver (9) comprises a drive chassis (10) with a plunger rod (11) and a locked drive spring (13), wherein the locked drive spring (13) is acting between the housing (3) and the drive chassis (10) for generating the force necessary to move the plunger (7) for dispensing the drug; and a needle guard (15) provided axially movable within the housing (3) and configured to release the locked drive spring (13) of the driver (9) for dispensing the drug upon axial movement.

A third aspect of the present invention refers to the electronic module (27) or autoinjector (1) of any one of the first and second aspect,
wherein the electronic module (27) comprises a detection unit and a notification unit,
wherein the detection unit is configured to monitor, i.e. surveil one or more moving parts of the autoinjector (1) and to generate information about a administering process of the autoinjector (1) or parameters related thereto based on an operating state of at least one component of the autoinjector (1);
wherein the notification unit is configured to generate and to output a notification signal including or indicating the generated information about the administering process or the parameters related thereto.

A fourth aspect of the present invention refers to the electronic module (27) or autoinjector (1) of the third aspect,
wherein the detection unit comprises a photo-reflector sensor (29) configured to monitor the movement of at least one component of the autoinjector (1), in particular of a drive chassis (10) of the autoinjector (1), during the administering process.

A fifth aspect of the present invention refers to the electronic module (27) or autoinjector (1) of the fourth aspect,
wherein the drive chassis (10) comprises at least one optical pattern (12) which is monitored by the photo-reflector sensor (29) for monitoring the dispensing movement of the plunger rod (11).

A sixth aspect of the present invention refers to the electronic module (27) or autoinjector (1) of the third aspect,
wherein the detection unit comprises a time of flight sensor (39) configured to monitor the movement of at least one component of the autoinjector (1), in particular of a plunger rod (11) of the autoinjector (1), during the administering process.

A seventh aspect of the present invention refers to the electronic module (27) or autoinjector (1) of the sixth aspect,
wherein the time of flight sensor (39) is an optical time of flight sensor and the plunger rod (11) comprises a reflective surface which is monitored by the optical time of flight sensor (39) for monitoring the dispensing movement of the plunger rod (11).

An eight aspect of the present invention refers to the electronic module (27) or autoinjector (1) of the third aspect,
wherein the detection unit comprises a directional microphone (39), in particular a micro-electromechanical system (MEMS) microphone, configured to detect a signal sound generated by at least one component of the autoinjector (1), in particular of a drive chassis (10) of the autoinjector (1), during the administering process.

A ninth aspect of the present invention refers to the electronic module (27) or autoinjector (1) of the eight aspect,
wherein the drive chassis (10) comprises at least one acoustic active section (25), in particular a clicker protrusion, generating an acoustic signal upon movement of the drive chassis (10) in the proximal direction, such that the directional microphone (39) can monitor the movement of the plunger rod (11) via the generated acoustic signal.

A tenth aspect of the present invention refers to the electronic module (27) or autoinjector (1) of the third aspect,
wherein the detection unit comprises at least one frangible circuit element (43) configured to be broken when at least one component of the autoinjector (1), in particular a drive chassis (10) of the autoinjector (1), reaches a specific position during the administering process, to monitor the administering process.

An eleventh aspect of the present invention refers to the electronic module (27) or autoinjector (1) of any one of the third to tenth aspect,
wherein the notification unit comprises an active wireless communicator (35) configured to transmit information actively wirelessly to a user device like a smart phone (59) or similar.

A twelfth aspect of the present invention refers to the electronic module (27) or autoinjector (1) of the eleventh aspect,
wherein the active wireless communication is based on the "cellular" communication technology SigFox.

A thirteenth aspect of the present invention refers to the electronic module (27) or autoinjector (1) of the eleventh aspect,
wherein the active wireless communication is based on the "cellular" communication technology NB-IoT.

A fourteenth aspect of the present invention refers to the electronic module (27) or autoinjector (1) of the eleventh aspect,
wherein the active wireless communication is based on Bluetooth.

A fifteenth aspect of the present invention refers to the electronic module (27) or autoinjector (1) of any one of the third to tenth aspect,
wherein the notification unit comprises a passive wireless communicator (41) configured to transmit information passively wirelessly to a user device like a smartphone (59) or similar.

A sixteenth aspect of the present invention refers to the electronic module (27) or autoinjector (1) of the fifteenth aspect,
wherein the passive wireless communication is based on NFC (near-field communication), in particular with an RFID chip (45) and an antenna (47).

A seventeenth aspect of the present invention refers to the electronic module (27) or autoinjector (1) of any one of the third to sixteenth aspect,
wherein the notification unit comprises an optical notifier configured to directly display information to a user of the autoinjector (1).

An eighteenth aspect of the present invention refers to the electronic module (27) or autoinjector (1) of the seventeenth aspect,
wherein the optical notifier includes at least one, in particular several, notification LED.

A nineteenth aspect of the present invention refers to the electronic module (27) or autoinjector (1) of any one of the third to fourteenth, seventeenth and eighteenth aspect,
wherein the electronic module (27) comprises a micro-controller (31) and a power supply (33),
wherein the micro-controller (31) is configured to receive the information about the administering process from the detection unit, to process the receive information and to control the operation of the notification unit to output a user feedback based on the processed information.

A twentieth aspect of the present invention refers to the electronic module (27) or autoinjector (1) of any one of the third to fourteenth and seventeenth to nineteenth aspect,
wherein the electronic module (27) comprises a, in particular mechanical, wake switch (37),
wherein the wake switch (37) is coupled to the micro-controller (31) and configured to switch the operation state of the micro-controller (31) between an ON state and an OFF state.

A twenty-first aspect of the present invention refers to the electronic module (27) or autoinjector (1) of the twentieth aspect,
wherein the wake switch (37) is configured not to be operated by a user of the autoinjector (1) but automatically during the usage of the autoinjector (1), in particular either by the needle guard (15) or the drive chassis (10) with the plunger rod (11).

A twenty-second aspect of the present invention refers to the electronic module (27) or autoinjector (1) of any one of the preceding aspects,
wherein the electronic module (27) is provided as integral component of the autoinjector (1) non-releasably coupled to the components of the autoinjector (1).

A twenty-third aspect of the present invention refers to the electronic module (27) or autoinjector (1) of the twenty-second aspect,
wherein the components of the electronic module (27) are positioned within a/the housing (3) of the autoinjector (1).

A twenty-fourth aspect of the present invention refers to the electronic module (27) or autoinjector (1) of the twenty-second or twenty-third aspect,
wherein the electronic module (27) is positioned side by side with the components of the autoinjector (1).

A twenty-fifth aspect of the present invention refers to The electronic module (27) or autoinjector (1) of any one of the first to twenty-first aspect,
wherein the electronic module (27) is provided as separate add-on module configured to be coupled releasably to the autoinjector (1).

A twenty-sixth aspect of the present invention refers to the electronic module (27) or autoinjector (1) of the twenty-fifth aspect,
wherein the electronic module (27) comprises a sealing membrane (51) positioned between the electronic module (27) and the autoinjector (1) in a state in which the electronic module (27) is coupled to the autoinjector (1).

A twenty-seventh aspect of the present invention refers to the electronic module (27) or autoinjector (1) of the twenty-sixth aspect,
wherein the sealing membrane (51) is made of material that is transparent for specific light, in particular IR light, such that an optical time of flight sensor (39) can be used for monitoring the administering process through the sealing membrane (51).

A twenty-eighth aspect of the present invention refers to the electronic module (27) or autoinjector (1) of any one of the twenty-fifth to twenty-seventh aspect,
wherein the electronic module (27) comprises an attachment switch (49) configured to detect the attachment of the electronic module (27) to the autoinjector (1).

A twenty-ninth aspect of the present invention refers to the electronic module (27) or autoinjector (1) of any one of the preceding twenty-fifth to twenty-eighth aspect, wherein the autoinjector (1) is provided with an NFC tag (57) containing information about the drug stored within the autoinjector (1).

A thirtieth aspect of the present invention refers to the electronic module (27) or autoinjector (1) of the twenty-ninth aspect with the features any one of the twenty-fifth to twenty-eighth aspect,
wherein the electronic module (27) comprises an NFC reader (63) configured to automatically read the information stored within the NFC tag (57).

A thirty-first aspect of the present invention refers to the electronic module (27) or autoinjector (1) of any one of the preceding aspects,
wherein at least one component of the autoinjector (1) comprises a barcode or QR code (61) containing information about the drug within the autoinjector (1) and configured to be read by a user device like a smartphone (59).

A thirty-second aspect of the present invention refers to the electronic module (27) or autoinjector (1) of any one of the preceding aspects with the features of any one of the twenty-firth to twenty-eighth and thirtieth aspect,
wherein the electronic module (27) comprises a mechanical coding feature (65) only allowing the attachment of the electronic module (27) to autoinjectors (1) provided with a matching mechanical coding feature.

A thirty-third aspect of the present invention refers to the electronic module (27) or autoinjector (1) of any one of the preceding third to twenty-third and twenty-firth to thirty-second aspect not having the further features of the twenty-fourth aspect, wherein the electronic module (27) is configured to be or is positioned at a distal end of the autoinjector (1) axially behind the components of the autoinjector (1).

A thirty-fourth aspect of the present invention refers to the electronic module (27) or autoinjector (1) of any one of the preceding aspects,
wherein the autoinjector (1) is a single use fixed dose autoinjector.

A thirty-fifth aspect of the present invention refers to the electronic module (27) or autoinjector (1) of any one of the preceding first to thirty-third aspect,
wherein the autoinjector (1) is a single use variable dose autoinjector.

A thirty-sixth aspect of the present invention refers to the electronic module (27) or autoinjector (1) of any one of the preceding first to thirty-third aspect,
wherein the autoinjector (1) is a multiple use fixed dose autoinjector.

A thirty-seventh aspect of the present invention refers to the electronic module (27) or autoinjector (1) of any one of the preceding first to thirty-third aspect, wherein the autoinjector (1) is a multiple use variable dose autoinjector.

A thirty-eighth aspect of the present invention refers to the electronic module (27) or autoinjector (1) of any one of the preceding aspects,
wherein the electronic module (27) is configured to detect and to monitor the dose administering process with the autoinjector (1) and/or parameters related thereto based on a movement status of one or more moving parts of the autoinjector (1) or based on a position of one or more moving parts of the autoinjector (1).

A thirty-ninth aspect of the present invention refers to the electronic module (27) or autoinjector (1) of any one of the preceding first to thirty-seventh aspect,
wherein the electronic module (27) is configured to detect and to monitor the dose administering process with the autoinjector (1) and/or parameters related thereto based on a movement status and based on a position of one or more moving parts of the autoinjector (1).

A fortieth aspect of the present invention refers to the electronic module (27) or autoinjector (1) of the thirty-ninth aspect,
wherein the electronic module (27) is configured to detect and to monitor the dose administering process with the autoinjector (1) and/or parameters related thereto based on the movement status and the position of one single moving part, in particular a drive chassis (10), of the autoinjector (1).

A forty-first aspect of the present invention refers to the electronic module (27) or autoinjector (1) of any one of the preceding aspects,
wherein the micro-controller waits a specific period of time before finally determining the end of the dose administering process after no further change in anyone of the monitored parameters is changing any more.

A forty-second aspect of the present invention refers to the electronic module (27) or autoinjector (1) of the forty-first aspect,
wherein the specific period of time is 1, 2, 3, 5 or 10 seconds.

A forty-third aspect of the present invention refers to the electronic module (27) or autoinjector (1) of any one of the preceding aspects,
wherein an outer contour of the autoinjector (1) together with the electronic module (27) is flush seen along the longitudinal axis of the autoinjector (1).

A forty-fourth aspect of the present invention refers to the electronic module (27) or autoinjector (1) of any one of the preceding aspects,
wherein the electronic module (27) is configured to monitor the movement of a part of the drive chassis (10) during the administering process.

### Reference numeral list

- 1: autoinjector
- 3: housing
- 3a: outer body
- 3b: inner body
- 5: cartridge/ syringe
- 7: plunger
- 9: driver
- 10: drive chassis
- 11: plunger rod
- 11a: reflective surface
- 12: optical pattern
- 13: drive spring
- 15: needle guard
- 16: needle guard spring
- 17: cap
- 19: needle shield
- 21: needle assembly
- 22: needle
- 23: locking element
- 25: resilient feature/acoustically active section
- 26: end of dose click protrusion
- 27: electronic module
- 28: PCBA
- 29: photo-reflector sensor
- 31: micro-controller
- 33: power supply
- 35: active wireless communicator
- 37: wake switch
- 39: time of flight sensor/ directional microphone
- 41: NFC connectivity system
- 43: frangible circuit element
- 45: RFID chip
- 47: antenna
- 49: attachment switch
- 51: sealing member
- 53: clip feature
- 55: protrusion
- 57: NFC tag
- 59: smartphone
- 61: barcode/ QR code
- 63: NFC reader
- 65: mechanical coding feature
- 67: window

## Claims

1. An electronic module (27) connectable to an autoinjector (1) or an autoinjector (1) with such an electronic module (27), wherein the electronic module (27) is configured to detect and to monitor a dose administering process with the autoinjector (1) and/or parameters related thereto and to provide a user of the autoinjector (1) with feedback on the conducted dose administering process and/or parameters related thereto.

2. The electronic module (27) or autoinjector (1) of claim 1,
wherein the electronic module (27) comprises a photo-reflector sensor (29) configured to monitor the movement of at least one component of the autoinjector (1), in particular of a drive chassis (10) of the autoinjector (1), during the administering process,
wherein in particular the drive chassis (10) comprises at least one optical pattern (12), which is monitored by the photo-reflector sensor (29) for monitoring the dispensing movement of the plunger rod (11).

3. The electronic module (27) or autoinjector (1) of claim 1 or 2,
wherein the electronic module (27) comprises a time of flight sensor (39) configured to monitor the movement of at least one component of the autoinjector (1), in particular of a plunger rod (11) of the autoinjector (1), optionally wherein the plunger rod (11) is a part of a drive chassis (10), during the administering process,
wherein in particular the time of flight sensor (39) is an optical time of flight sensor and the plunger rod (11) comprises a reflective surface which is monitored by the optical time of flight sensor (39) for monitoring the dispensing movement of the plunger rod (11).

4. The electronic module (27) or autoinjector (1) of claim 1 or 2,
wherein the electronic module (27) comprises a directional microphone (39), in particular a micro-electromechanical system (MEMS) microphone, configured to detect a signal sound generated by at least one component of the autoinjector (1), in particular of a drive chassis (10) of the autoinjector (1), during the administering process,
wherein in particular the drive chassis (10) comprises at least one acoustically active section (25), in particular a clicker protrusion, generating an acoustic signal upon movement of the drive chassis (10) in the proximal direction, such that the directional microphone (39) can monitor the movement of the plunger rod (11) via the generated acoustic signal.

5. The electronic module (27) or autoinjector (1) of claim 1 or 2,
wherein the electronic module (27) comprises at least one frangible circuit element (43) configured to be broken when at least one component of the autoinjector (1), in particular a drive chassis (10) of the autoinjector (1), reaches a specific position during the administering process, to monitor the administering process.

6. The electronic module (27) or autoinjector (1) of any one of the preceding claims,
wherein the electronic module (27) comprises an active wireless communicator (35) configured to transmit information actively wirelessly to a user device like a smart phone (59) or similar,
wherein in particular the active wireless communication is based on the "cellular" communication technology SigFox, on the "cellular" communication technology NB-IoT or on Bluetooth.

7. The electronic module (27) or autoinjector (1) of any one of the preceding claims,
wherein the electronic module (27) comprises a passive wireless communicator (41) configured to transmit information passively wirelessly to a user device like a smartphone (59) or similar,
wherein in particular the passive wireless communication is based on NFC (near-field communication), in particular with an RFID chip (45) and an antenna (47).

8. The electronic module (27) or autoinjector (1) of any one of the preceding claims,
wherein the electronic module (27) comprises an optical notifier configured to directly display information to a user of the autoinjector (1),
wherein in particular the optical notifier includes at least one, in particular several, notification LED.

9. The electronic module (27) or autoinjector (1) of any one of the preceding claims,
wherein the electronic module (27) comprises a micro-controller (31) and a power supply (33),
wherein the micro-controller (31) is configured to receive the information about the administering process, to process the receive information and to control the output of user feedback based on the processed information.

10. The electronic module (27) or autoinjector (1) of any one of the preceding claims,
wherein the electronic module (27) comprises a, in particular mechanical, wake switch (37),
wherein the wake switch (37) is coupled to the micro-controller (31) and configured to switch the operation state of the micro-controller (31) between an ON state and an OFF state,
wherein in particular the wake switch (37) is configured not to be operated by a user of the autoinjector (1) but automatically during the usage of the autoinjector (1), in particular either by the needle guard (15) or the drive chassis (10) with the plunger rod (11).

11. The electronic module (27) or autoinjector (1) of any one of the preceding claims,
wherein the electronic module (27) is provided as integral component of the autoinjector (1) non-releasably coupled to the components of the autoinjector (1),
wherein in particular the components of the electronic module (27) are positioned within a/the housing (3) of the autoinjector (1),
wherein preferably the electronic module (27) is positioned side by side with the components of the autoinjector (1) or behind them in a distal end of the housing 3.

12. The electronic module (27) or autoinjector (1) of any one of claims 1 to 10, wherein the electronic module (27) is provided as separate add-on module configured to be coupled releasably to the autoinjector (1),
wherein in particular the electronic module (27) comprises a sealing membrane (51) positioned between the electronic module (27) and the autoinjector (1) in a state in which the electronic module (27) is coupled to the autoinjector (1),
wherein preferably the sealing membrane (51) is made of material that is transparent for specific light, in particular IR light, such that an optical time of flight sensor (39) can be used for monitoring the administering process through the sealing membrane (51).

13. The electronic module (27) or autoinjector (1) of claim 12,
wherein the electronic module (27) comprises an attachment switch (49) configured to detect the attachment of the electronic module (27) to the autoinjector (1),
wherein in particular the autoinjector (1) is provided with an NFC tag (57) containing information about the drug stored within the autoinjector (1), wherein preferably the electronic module (27) comprises an NFC reader (63) configured to automatically read the information stored within the NFC tag (57); and/or
wherein at least one component of the autoinjector (1) comprises a barcode or QR code (61) containing information about the drug within the autoinjector (1) and configured to be read by a user device like a smartphone (59); and/or
wherein the electronic module (27) comprises a mechanical coding feature (65) only allowing the attachment of the electronic module (27) to autoinjectors (1) provided with a matching mechanical coding feature.

14. The electronic module (27) or autoinjector (1) of any one of the preceding claims,
wherein the autoinjector (1) is preferably a single use fixed dose autoinjector or one of a single use variable dose autoinjector, a multiple use fixed dose autoinjector and a multiple use variable dose autoinjector.

15. The electronic module (27) or autoinjector (1) of any one of the preceding claims,
wherein the electronic module (27) is configured to detect and to monitor the dose administering process with the autoinjector (1) and/or parameters related thereto based on at least one of, preferably based on both of, a movement status of one or more moving parts of the autoinjector (1) and a position of one or more moving parts of the autoinjector (1),
wherein in particular the electronic module (27) is configured to detect and to monitor the dose administering process with the autoinjector (1) and/or parameters related thereto based on movement and/or position information of one single moving part of the autoinjector (1), in particular of a drive chassis (10) thereof.
